(19)

Europäisches Patentamt
European Patent Office
Office européen des brevets

(11) **EP 3 946 197 B1**

(12) **EUROPEAN PATENT SPECIFICATION**

(45) Date of publication and mention of the grant of the patent:
**10.12.2025 Bulletin 2025/50**

(21) Application number: **20721000.6**

(22) Date of filing: **27.03.2020**

(51) International Patent Classification (IPC):
*A61F 13/537* (2006.01)    *A61F 13/538* (2006.01)
*A61F 13/532* (2006.01)

(52) Cooperative Patent Classification (CPC):
**A61F 13/532**

(86) International application number:
**PCT/US2020/025146**

(87) International publication number:
**WO 2020/205485 (08.10.2020 Gazette 2020/41)**

(54) **A NONWOVEN SUITABLE FOR USE IN ABSORBENT ARTICLE**

VLIESSTOFF FÜR SAUGFÄHIGEN ARTIKEL

NON-TISSÉ APPROPRIÉ POUR ÊTRE UTILISÉ DANS UN ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2019   US 201962826214 P**

(43) Date of publication of application:
**09.02.2022   Bulletin 2022/06**

(73) Proprietor: **The Procter & Gamble Company Cincinnati, OH 45202 (US)**

(72) Inventors:
- **PERI, Andrea**
  **65824 Schwalbach am Taunus (DE)**
- **VIENS, Gerard, Alain**
  **Cincinnati, Ohio 45202 (US)**
- **HECKER, Rolf**
  **65824 Schwalbach am Taunus (DE)**
- **NARANJOMARTIN, Carlos, Domingo**
  **65824 Schwalbach am Taunus (DE)**
- **HIPPE, Matthias, Konrad**
  **65824 Schwalbach am Taunus (DE)**
- **GRAY, Brian, Francis**
  **Cincinnati, Ohio 45202 (US)**
- **BELLUCCI, Remo**
  **65824 Schwalbach am Taunus (DE)**
- **GIOVANNI, Sara, Lyn**
  **Cincinnati, Ohio 45202 (US)**

(74) Representative: **P&G Patent Germany Procter & Gamble Service GmbH Sulzbacher Straße 40 65824 Schwalbach am Taunus (DE)**

(56) References cited:
**US-A1- 2014 343 523     US-A1- 2018 098 889**

**Description**

FIELD OF THE INVENTION

The present disclosure generally relates to a nonwoven that can be used in a disposable absorbent article for personal hygiene such as a diaper, a feminine care pad or an adult incontinence product. The nonwoven is particularly useful to be used as an acquisition layer between the topsheet and the absorbent core of the article.

BACKGROUND OF THE INVENTION

Disposable absorbent articles such as feminine hygiene products, taped diapers, pant-type diapers and incontinence products are designed to absorb fluids from the wearer's body. There is a continuous need to provide absorbent articles that have good absorbency, feel soft to touch, and are economical to produce.

The perception of softness, or haptic, of absorbent articles depend of multiple factors that need to be satisfied at the same time. For example, a diaper needs to have enough cushiness when compressed so that it provides padding to the delicate genital area, but at the same time it needs to be flexible to wrap around the wearer during motion like a textile. These properties are however in general in contradiction, in other words when cushiness is increased (for example by increasing the amount of soft material used), the flexibility is often compromised. There is therefore a need to find a material which is at the same time cushy and highly flexible, while being economical to produce for disposable absorbent articles. Such material may be particularly useful as an acquisition layer to deliver the required softness perception while maintaining an adequate level of acquisition performance.

US2018/0,098,889A1 and US2014/0,343,523A1 disclose secondary topsheet for femcare pads made of spunlace material. The purpose of these secondary topsheet is to provide increased stiffness in CD of the femcare pads. The nonwovens of the invention achieve different properties, i.e. high drapability and cushiness.

SUMMARY OF THE INVENTION

The invention is, in a first aspect, for a nonwoven suitable for use in an absorbent article, as defined in the claims. The nonwoven has the following properties:

- a Horizontal Bending Drop at 100 mm of at least 75 mm; and
- a Z-Compliance Index of at least 50 mm³/N.

Further advantageous features for the nonwoven are as indicated in the dependent claims and further described in this description.

In a second aspect, the invention is for an absorbent article comprising a nonwoven according to the invention. The nonwoven may in particular be used as an acquisition layer in an absorbent article such as a diaper.

BRIEF DESCRIPTION OF THE DRAWINGS

Fig. 1 shows a schematic setup for conducting the Horizontal Bending Drop Test;
Fig. 2 shows a schematic production process for an integrated nonwoven comprising three strata;
Fig. 3 shows a schematic diaper, as seen from the top, with some layers partially removed;
Fig. 4 shows a schematic cross-section of the diaper of Fig. 3.

DETAILED DESCRIPTION OF THE INVENTION

**Definitions**

The term "nonwoven" as used herein refers to a manufactured material, web, sheet or batt of directionally or randomly oriented fibers, bonded by friction, and/or cohesion and/or adhesion, excluding paper and products which are woven, knitted, tufted, stitch-bonded, incorporating binding yarns or filaments, or felted by wet milling, whether or not additionally needled. The fibers may be of natural or man-made origin. The fibers may be staple or continuous filaments or be formed in situ. The porous, fibrous structure of a nonwoven may be configured to be liquid permeable or impermeable, as desired.

The term "spunlace nonwoven" means a nonwoven wherein the cohesion and the interlacing of the fibers with one another is obtained by means of a plurality of jets of water under pressure passing through a moving fleece or cloth and, like needles, causing the fibers to intermingle with one another. These spunlace nonwovens are essentially defined by the fact that their consolidation results from hydraulic interlacing. "Spunlace nonwoven", as used herein, also relates to a nonwoven formed of two or more webs (stratum), which are combined with each other by hydraulic interlacing. The two webs, prior to being combined into one nonwoven by hydraulic interlacing, may have underdone bonding processes, such as heat and/or pressure bonding by using e.g. a patterned calendar roll and an anvil roll to impart a bonding pattern. However, the two webs are combined with each other solely by hydraulic interlacing.

"Absorbent article" refers to wearable devices, which absorb and/or contain liquid, and more specifically, refers to devices, which are placed against or in proximity to the body of the wearer to absorb and contain the various exudates discharged from the body. Absorbent articles considered include baby diapers, training pants, adult incontinence undergarments (e.g., liners, pads and briefs) and/or feminine hygiene products.

"Machine Direction" or "MD" as used herein means the direction parallel to the flow of the nonwoven through the nonwoven making machine and/or absorbent article manufacturing equipment.

"Cross-Machine Direction" or "CD" as used herein means the direction parallel to the width of the nonwoven making machine and/or absorbent article manufacturing equipment and perpendicular to the machine direction.

The "Z-direction" is orthogonal to both the Machine Direction and the Cross-Machine Direction.

## General description of a nonwoven according to the invention

The nonwovens of the present invention have a Horizontal Bending Drop at 100 mm (HBD@100mm) of at least 75 mm and a Z-Compliance Index of at least 50 mm$^3$/N. These properties are measured as indicated in the measurement method described further below.

The HBD@100mm may be advantageously at least 80 mm, and even more advantageously at least 85 mm. The maximum theoretical value for the HBD@100mm is 100 mm, but as indicated in the background having increased flexibility is difficult to combine with cushiness, as measured by the Z-Compliance Index. The HBD@100mm may thus typically be up to 99 mm, or up to 98 mm or even up to 96 mm. A nonwoven according to the present invention may thus have a HBD@100mm ranging of from 75 mm up to 99 mm, or from 80 mm up to 98 mm, or from 80 mm up to 97 mm.

The Z-Compliance Index measures the cushiness of the nonwoven. The nonwoven of the invention has a Z-Compliance Index of at least 50 mm$^3$/N. Advantageously, the Z-Compliance Index of the nonwoven may be of at least 55 mm$^3$/N, or at least 60 mm$^3$/N, or at least 65 mm$^3$/N, or at least 70 mm$^3$/N. There is no theoretical maximum value for the Z-Compliance Index, but in practice in order to reach a good compromise between cushiness and flexibility, the Z-Compliance Index of the nonwoven according to the invention may be up to 120 mm$^3$/N, or up to 110 mm$^3$/N, or up to 100 mm$^3$/N, or up to 90 mm$^3$/N.

The nonwoven of the invention may advantageously have a thickness and basis weight that also define a good compromise for the desired properties. A lower basis weight material and/or thinner material may be more economical and more bendable, but may have less cushiness, than the same material at higher basis weight and/or thicker material. Thus, the nonwoven of the invention may have a thickness (also named "caliper") of from 0.50 mm to 4.00 mm, in particular of from 1.00 mm to 3.00 mm, as measured at a pressure of 0.85 kPa. This caliper is designated as C1 and is measured during the Z-Compliance Index and % Recovery Measurement Method, described further below. The nonwovens of the invention have a basis weight ranging of from about 40 grams per square meter (gsm) to about 200 gsm, in particular from 60 gsm to 150 gsm, or from 70 gsm to 120 gsm.

The nonwovens of the invention may further a Percent Recovery of at least 50%, or at least 60%, or at least 65%, or at least 70%, as measured by the Z-Compliance Index and Percent Recovery Measurement Method described herein. The theoretical maximum Percent Recovery value is 100%. In order to find a good compromise between cushiness and flexibility, suitable nonwoven may have Percent Recovery of up to 95%, or up to 90% or even up to 85%. Suitable Percent Recovery for the nonwoven of the invention may thus range of from 50% up to 95%, or from 60% to 90%, of from 65% up to 85%.

The present invention may be embodied by a wide variety of nonwovens suitable for use in absorbent article having the required properties. A particular type of suitable nonwoven are integrated nonwovens. Integrated nonwovens comprise fibers which have been integrated. Fiber integration of a nonwoven can occur via any suitable process which entangles fibers primarily in a Z-direction (positive or negative). Exemplary processes which are amenable in creating such fiber integration include needlepunching and spunlacing. Needlepunching involves the mechanical interlocking of fibers of a spunbonded and/or carded web(s). In the needlepunching process, a plurality of barbed needles repeatedly pass in and out of nonwoven web(s) and push fibers of the nonwoven web(s) in a positive and/or negative Z-direction. In contrast, the spunlace process uses high-speed jets of water to cause the interlocking of fibers of a nonwoven web(s). The high-speed jets of water push fibers of the nonwoven web in the positive or negative Z-direction. With the aid of a microscope, needlepunched nonwoven materials comprise a plurality of discrete Z-direction fiber integrations in both MD and CD directions, while spunlace nonwovens generally comprise much more continuous integrations along the MD direction but discrete in the CD direction.

The nonwoven of the present invention has a first surface and an opposing second surface. The nonwoven of the present invention may in particular comprise two, three or more strata along the Z-direction which have been integrated as described above between these two surfaces. These strata are typically carded webs made of staple fibers.

Due to the fiber integration, the nonwoven may not require adhesives or latex binders for stability. Additionally, a carded staple fiber nonwoven can be manufactured from an assortment of suitable fiber types that produce the desired performance characteristics. In particular, the nonwovens of the invention comprise a combination of absorbent fibers, stiffening fibers and resilient fibers.

In order to enhance the stabilizing effect of the integration, one or more of these fibers may be crimped prior to integration. For example, where synthetic fibers are utilized, these fibers may be mechanically crimped via intermeshing teeth. And for the absorbent fibers, these fibers may be mechanically crimped and/or may have a chemically induced crimp due to the variable skin thickness formed during creation of the absorbent fibers.

Overall, the nonwovens of the invention comprise from about 20 percent to about 75 percent by weight, in particular from about 25 percent to about 60 percent by weight, or from about 30 percent to about 50 percent by weight, specifically including any values within these ranges and any ranges created thereby, of absorbent fibers. In one specific example, the nonwoven may comprise about 50 percent by weight absorbent fibers.

Overall, the nonwovens of the invention comprise from about 1 percent to about 50 percent, in particular from about 10 percent to about 40 percent, or from about 20 percent to about 30 percent, of stiffening fibers, specifically reciting all values within these ranges and any ranges created thereby. In one specific example, the nonwoven may comprise about 20 percent by weight stiffening fibers.

Overall, the nonwovens of the present invention comprise from about 10 percent to about 50 percent, in particular from about 13 percent to about 40 percent, or from about 20 percent to about 35 percent, or from about 25 percent to about 30 percent by weight, of resilient fibers, specifically reciting all values within these ranges and any ranges created thereby. In one specific example, the nonwoven of the invention may comprise about 30 percent by weight resilient fibers.

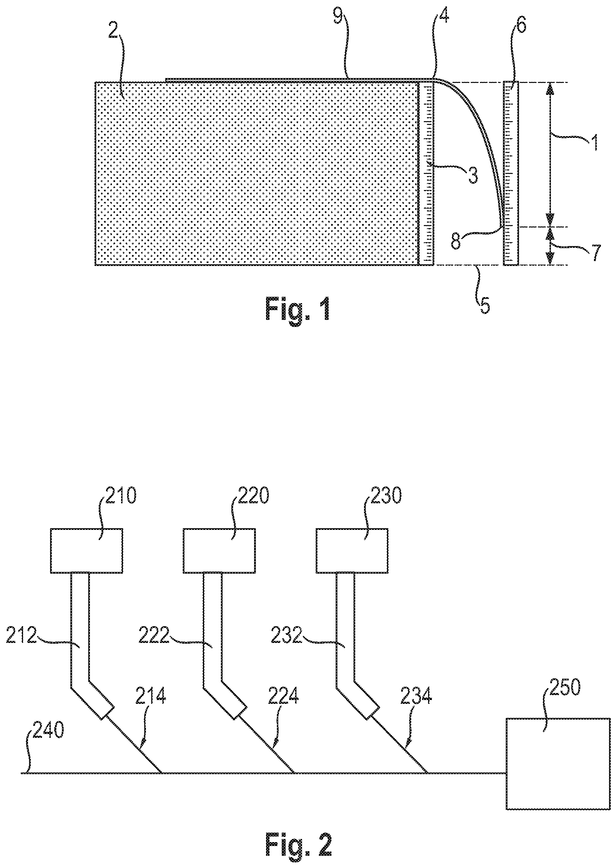

## Process for making an integrated nonwoven (as illustrated in Fig. 2)

A schematic representation of a carding and integrating process suitable for creating an integrated nonwoven according to the present disclosure is provided in Fig. 2. As shown, a plurality of carding machines 210, 220, and 230 may each form a carded web (which are designed as "stratum" in the integrated nonwoven), e.g. 214, 224, and 234, respectively, which is transferred to a carrier belt 240. Each of the carded nonwoven webs 214, 224, and 234, may be provided to the carrier belt 240 via a web chute 212, 222, 232, respectively. It is also worth noting that after the carded nonwoven 214 is deposited on the carrier belt 240, the carded nonwoven 224 is then deposited on the first carded nonwoven 214 on the carrier belt 240. Similarly, the third carded nonwoven web 234 deposited on the second carded nonwoven 224 and the first carded nonwoven 214 on the carrier belt 240. Subsequently, each of the first, second, and third carded nonwoven webs 214, 224, and 234 are then provided to an integration process 250 which utilizes either needles and/or high pressure water streams to entangle the fibers of the first, second, and third carded nonwoven webs providing an integrated nonwoven 260. Both carding and integration processes are well known in the art.

It is worth noting that with the arrangement provided in schematic diagram of FIG. 2, a wide variety of configurations for a nonwoven may be achieved. However, it is advantageous that the nonwoven of the present disclosure has adequate openness to allow for quick acquisition of fluid. With this in mind, the carded webs, i.e. 214, 224, and/or 234, may be different from one another. For example, one of the carded webs may comprise a different fiber blend than the others. Specifically, assuming the first carded web would be closest to the wearer-facing surface in an absorbent article, the fiber selection for the first carded web 214 may be such that there is more openness associated with this web. The second carded web 224 may be similarly configured.

If the first carded web 214 and the second carded web 224 comprise the same fiber blend, then the fluid management layers of the present disclosure may be processed via two cards instead of three. However, such processing would potentially slow the production of the first carded layer 214 which would comprise a much higher basis weight than that of the third carded web 234.

The first carded nonwoven 214, the second carded nonwoven 224 (optional, as previously mentioned), and the third carded nonwoven 234 are integrated. Once they are integrated, they cannot be manually separated - at least not without substantial effort and time. Each carded web forms a stratum in the overall integrated nonwoven layer. Each stratum can maintain its unique properties for at least a portion of the stratum along the z-direction, even when integrated into a larger nonwoven. The nonwoven when used as acquisition layer 54 provides capillary suction to "pull" fluid through the topsheet, which is competing for trickle/low flow conditions. The nonwoven also can contain a gush by providing distribution functions to efficiently utilize the absorbent core, as well as provide intermediate storage until the absorbent core can accept fluid.

The nonwoven of the invention advantageously has the ability to acquire liquid insults from the topsheet and to pull the liquid far enough from the topsheet, such that the topsheet does not feel wet. To accomplish this, the inventors have found that while absorbent fibers adjacent to the topsheet can help pull liquid from the topsheet, too many absorbent fibers can lead to a wet feeling topsheet. As such, the amount of absorbent fibers in the stratum closest to the topsheet is important to control. Namely too many absorbent fibers in the first carded nonwoven web 214 and/or second carded nonwoven web 224, can lead to a wet feeling topsheet 20 (assuming the first carded nonwoven 214 and the second carded nonwoven 224 are more proximal to the topsheet 20 than the third carded nonwoven 234). In the following, the first stratum is considered to be on the side of the nonwoven intended to be placed closest to the topsheet, and the third stratum on the side of the nonwoven closest to the absorbent core, when the integrated nonwoven is used as an acquisition layer.

The first stratum 214 (corresponding to first carded nonwoven 214) may comprise absorbent fibers, stiffening fibers, and resilient fibers. To achieve sufficient void volume, the first stratum 214 may comprise from about 10 percent to about 50 percent, or from about 12 percent to about 40 percent, or from about 15 percent to about 30 percent by weight, specifically reciting all values within these ranges and any ranges created thereby, of absorbent fibers. The first stratum 214 may further comprise from about 20 percent to about 75 percent, or from about 25 percent to about 60 percent, or from about 30 percent to about 45 percent by weight, specifically including all values within these ranges and any ranges created thereby of stiffening fibers. The first stratum 214 may further comprise from about 20 percent to about 75 percent, or from about 30 percent to about 60 percent, or from about 40 percent to about 50 percent by weight, specifically including all values within these ranges and any ranges created thereby of resilient fibers.

The second stratum 224 (corresponding to second carded nonwoven 224) may be constructed similar to the first stratum 214. Such a construction for the second stratum 224 would facilitate manufacturing to some extent. Recall also that the second stratum 224 is optional. However, as the second stratum 224 may be disposed more distal from the topsheet than the first stratum 214, void volume could be adjusted slightly downward.

Regarding the third stratum 234 (corresponding to third carded nonwoven 234), the configuration of this stratum can vary. Additionally, the third stratum 234 may have sufficient capability to acquire and distribute fluid which is within the void volume of the first stratum 214 and/or second stratum 224. To appropriately deliver the acquisition and distribution attributes desired, absorbent fibers can be utilized. So, the third stratum 234 may comprise from about 50 percent to about 100 percent, or from about 60 percent to about 90 percent, or from about 70 percent to about 80 percent by weight, specifically reciting all values within these ranges and any ranges created thereby, of absorbent fibers.

Regarding the stiffening fibers and resilient fibers in the third stratum 234, as noted the third layer should be provided with the ability to acquire and distribute fluid from the void volume of the first stratum 214 and the second stratum 224. As such, the amount of either stiffening fibers or resilient fibers should be carefully reviewed. As noted previously, neither stiffening and nor resilient fibers are required in the third stratum 234, e.g. 0 weight percent. With this in mind, the third stratum may comprise between 0 percent to about 30 percent, from about 5 percent to about 25 percent, or from about 10 percent to about 20 percent by weight, specifically including all values within these ranges and any ranges created thereby of stiffening and/or resilient fibers.

**Absorbent fibers**

Still referring to Fig. 2, any suitable diameter of absorbing fiber may be utilized. A suitable measure of diameter can be linked to linear density. For the first stratum 214 and/or second stratum 224, larger linear density values may be utilized as increased void volume can be desirable. For example, in the first stratum 214 and/or the second stratum 224 the absorbent fiber linear density may range from about 1 dtex to about 4 dtex, about 2.0 dtex to about 3.7 dtex, or from about 2.5 dtex to about 3.5 dtex, specifically reciting all values within these ranges and any ranges created thereby. In one specific example, the absorbent fiber may comprise a dtex of about 1.7 dtex.

However, for the third stratum 234, the linear density may need to be decreased. So, the linear density of the absorbent fibers in the third stratum 234 may range from about 1 dtex to about 3 dtex, about 1.4 dtex to about 2.7 dtex, or from about 1.7 dtex to about 2.0 dtex, specifically reciting all values within these ranges and any ranges created thereby. In one specific example, the absorbent fibers in the third stratum 234 may comprise a dtex of about 1.7.

While it is technically possible to have absorbent fibers within the integrated nonwoven that have varying linear densities, not all carding equipment may be suited to handle such variation between / among strata. To alleviate the possible processing issues, the absorbent fibers of the integrated nonwoven may comprise the same linear density throughout all the strata.

Any suitable absorbent fibers may be utilized. Some conventional absorbent fibers include cotton, rayon or regenerated cellulose or combinations thereof. In one example, the absorbent fibers may comprise viscose cellulose fibers. The absorbing fibers may comprise staple length fibers. The staple length of the absorbing fibers can be in the range of about 20 mm to about 100 mm, or about 30 mm to about 50 mm or about 35 mm to about 45 mm, specifically reciting all values within these ranges and any ranges created thereby.

The absorbent fibers may have any suitable shape. Some examples include trilobal, "H," "Y," "X," "T," or round. Further, the absorbing fibers can be solid, hollow or multi-hollow. Other examples of suitable multi-lobed, absorbent fibers for utilization in the carded staple fiber nonwovens detailed herein are disclosed in US6,333,108, US5,634,914 and US5,458,835 (all to Wilkes et al.). The trilobal shape can improve wicking and improve masking. Suitable trilobal rayon is available from Kelheim Fibres and sold under the trade name Galaxy. While each stratum may comprise a different shape of absorbing fiber, much like mentioned above, not all carding equipment may be suited to handle such variation between / among strata. In one specific example, the fluid management layer comprises round absorbent fibers.

**Stiffening fibers**

As noted previously, in addition to absorbent fibers, the nonwovens of the invention also comprise stiffening fibers. Stiffening fibers may be utilized to help provide structural integrity to the nonwoven. The stiffening fibers can help increase structural integrity of the nonwoven in a machine direction and in a cross machine direction which can facilitate web manipulation during processing of the nonwoven for incorporation into a disposable absorbent article. With that in mind, the constituent material of the stiffening fibers, the weight percentage of the stiffening fibers, and heat of processing should be carefully selected to still achieve the desired Horizontal Bending Drop at 100 mm values. Thus, while some stiffening is desirable, it should be controlled so that the nonwoven is still relatively very flexible compared to other application wherein bendability is not as importance, such as in feminine care pads. The heat stiffening process is discussed hereafter.

Any suitable stiffening fiber may be utilized. Some examples of suitable stiffening fibers include bi-component fibers comprising polyethylene and polyethylene terephthalate components or polyethylene terephthalate and co-polyethylene terephthalate components. The components of the bi-component fiber may be arranged in a core sheath arrangement, a side by side arrangement, an eccentric core sheath arrangement, a trilobal arrangement, or the like. In one specific example, the stiffening fibers may comprise bi-component fibers having polyethylene / polyethylene terephthalate components arranged in a concentric, core - sheath arrangement where the polyethylene is the sheath. As another example, mono-component fibers may be utilized, and the constituent material of the mono-component may comprise polypropylene or polylactic acid (PLA). It is worth noting that these components, e.g. polypropylene and polylactic acid can also be utilized in bi-component fibers as well.

The stiffening fibers can be polyethylene terephthalate (PET) fibers, or other suitable non-cellulosic fibers known in the art. The staple length of the stiffening fibers can be in the range of about 28 mm to about 100 mm, or in the range of about 37 mm to about 50 mm. Some carded staple fiber nonwovens include stiffening fibers with a staple length of about 38 mm to 42 mm. The PET fibers can have any suitable structure or shape. For example, the PET fibers can be round or have other shapes, such as spiral, scalloped oval, trilobal, scalloped ribbon, and so forth. Further, the PET fibers can be solid, hollow or multi-hollow. In some embodiments of the carded staple fiber nonwoven, the stiffening fibers may be fibers made of hollow/spiral PET. Optionally, the stiffening fibers may be spiral-crimped or flat-crimped. The stiffening fibers may have a crimp value of between about 4 and about 12 crimps per inch (cpi), or between about 4 and about 8 cpi, or between about 5 and about 7 cpi, or between about 9 and about 10 cpi. Particular non-limiting examples of stiffening fibers can be obtained from Wellman, Inc. Ireland under the trade names H1311 and T5974. Other examples of suitable stiffening fibers for utilization in the carded staple fiber nonwovens detailed herein are disclosed in US7,767,598 (Schneider et al.).

Other suitable examples of stiffening fibers include polyester/co-extruded polyester fibers. The stiffening fibers may be so-called bi-component fibers, where individual fibers are provided from different materials, usually a first and a second polymeric material. The two materials may be chemically different (hence the fibers are chemically heterogeneous) or they may differ only in their physical properties while being chemically identical (hence the fibers are chemically homogeneous). For example, the intrinsic viscosity of the two materials may be different, which has been found to influence the crimping behavior of the bi-component fibers. Bi-component fibers that are suitable as stiffening fibers are side-by-side bi-component fibers as disclosed for example in WO 99/00098. The stiffening fibers may also be a blend of bi-component fibers with polyester fibers.

With specific reference to bicomponent fibers comprised of a polypropylene/polyethylene fiber composition, in a cross-sectional view of a fiber, the material with a higher softening temperature can provide the central part (i.e., the core) of the fiber. The core typically is responsible for the bicomponent fiber's ability to transmit forces and have a certain rigidity or otherwise provide structures with resiliency. The outer coating on the core (i.e., the sheath) of the fiber can have a lower melting point and is used to facilitate thermally bonding of substrates comprising such fibers. In one embodiment, a polypropylene core is provided with a polyethylene coating on the outside, such that about 50%, by weight, of the fiber material is polypropylene and 50%, by weight, of the fiber material is polyethylene. Other quantitative amounts can of course be selected. For example, bicomponent fibers can have a composition from about 30% to about 70%, by weight, polyethylene, while others have about 35% to about 65%, by weigh polyethylene. In some embodiments, bicomponent fibers can have a composition from about 40% to about 60% or about 45% to about 55%, by weight, polyethylene.

Any suitable size of stiffening fibers may be utilized in the first stratum 214 and/or second stratum 224. Suitable linear densities of stiffening fiber may be from about 1.7 dtex to about 12 dtex, from about 4 dtex to about 10 dtex, or from about 5 dtex to about 7 dtex, specifically reciting all values within these ranges and any ranges created thereby. In one specific example, the stiffening fibers may comprise 5.8 dtex polyethylene terephthalate / polyethylene fibers for an incontinence article.

Similar to the absorbent fibers in the third stratum 234, stiffening fibers comprised by the third stratum 234 may comprise lower linear densities than those of their first and/or second stratum counterparts. Where the third stratum 234 comprises stiffening fibers, the linear density may range from about 0.9 dtex to about 6 dtex, from about 1.3 dtex to about 3 dtex, or from about 1.6 dtex to about 2.5 dtex, specifically reciting all values within these ranges or any ranges created thereby.

As noted previously, the nonwoven of the invention may be heat treated (heat stiffened). This heat treatment can create connection points amongst the stiffening fibers of the fluid management layer. So, where there is a higher percentage of stiffening fibers, more connection points may be created. Too many connection points can yield a much stiffer fluid management layer which may negatively impact comfort. As such, the weight percentage of the stiffening fibers may be kept relatively low to keep good bendability of the nonwoven.

With regard to the heat stiffening process, the temperature should be adapted to the constituent chemistry of the stiffening fibers and the desired properties of the resulting nonwoven. In general, the lower the temperature, the fewer stiffening fibers are molten to form bonds with each other, resulting in an overall less stiff nonwoven. According to the invention, a flexible, non-stiff nonwoven is desired, so that a relatively low temperature is advantageous for the type of stiffening fibers considered. For PE/PET bicomponent fibers, the temperature may thus be advantageously be kept under 125 degree Celsius, e.g. from 100 to 120 degree Celsius.

It is also worth noting, that in order to provide a uniform stiffness property across the nonwoven layer, any heating operation should be set up to provide uniform heating to the web. Even small variations in temperature can greatly impact the tensile strength of the fluid management layer.

## Resilient fibers

As noted previously, the nonwovens of the present disclosure additionally comprise resilient fibers. The resilient fibers help the nonwoven to maintain its permeability and cushion properties. Suitable fibers that may be utilized include in particular hollow fibers, spiral fibers, and/or hollow spiral fibers. For example, the resilient fibers can have a linear density of about 4 dtex to about 12 dtex, from about 6 dtex to about 11 dtex, or from about 8 dtex to about 10 dtex, specifically reciting all values within these ranges and any ranges created thereby. In one specific example, the resilient fibers may comprise a linear density of about 10 dtex hollow spiral polyethylene terephthalate fibers. In another specific example, the resilient fibers may comprise 6.7 dtex round polyethylene terephthalate fibers.

It is worth noting, that if smaller fiber sizes are utilized, the resiliency of the fluid management layer would be expected to decrease. And, with the decreased size at the same weight percentage, a higher number of fibers per gram would equate to a decrease in permeability of the nonwoven.

As for the absorbent fibers in the third stratum, the resilient fibers comprised by the third stratum 234 may comprise lower linear densities than those of their first and/or second stratum counterparts. Where the third stratum 234 comprises resilient fibers, the linear density may range from about 0.9 dtex to about 12 dtex, from about 1.3 dtex to about 9 dtex, or from about 1.6 dtex to about 6 dtex, specifically reciting all values within these ranges or any ranges created thereby.

The resilient fibers can be any suitable thermoplastic fiber, such as polypropylene (PP), polyethylene terephthalate, or other suitable thermoplastic fibers known in the art. The staple length of the resilient fibers can be in the range of about 20 mm to about 100 mm, or about 30 mm to about 50 mm or about 35 mm to about 45 mm. The resilient fibers may have a circular cross section with a hollow space in the centre that is spiral crimped. It is preferred that 10-15% of the cross sectional area are hollow, more preferably 20-30% of the cross sectional area are hollow. Without wishing to be bound by theory, it is believed that the spiral crimping of fibers is beneficial for their liquid acquisition and distribution behaviour. It is assumed that the spiral crimp increases the void space in an acquisition member formed by such fibers.

Often, an absorbent article, when being worn, is exposed to a certain pressure exerted by the wearer, which potentially decreases the void space in the acquisition member. Having good permeability and sufficient void space available are important for good liquid distribution and transport. It is further believed that the bicomponent spiral-crimped fibers as described above are suitable to maintain sufficient void volume even when an acquisition member is exposed to pressure. Also, spiral-crimped fibers believed to provide for good permeability as for a given fiber dtex value, the hollow fiber cross-section allows for a larger outer diameter of the fiber as compared to a compact cross-section. The outer diameter of a fiber appears to determine the permeability behavior of an acquisition member formed by such fibers.

## Examples of nonwoven according to the invention

In the following, the abbreviation CV means viscose rayon fibers (absorbent fibers), PE/PET means bicomponent fibers having a PET core and a PE sheath structure (stiffening fibers), HS means Hollow Spiral fibers (resilient fibers).

Example 1:

Example 1 is a 110 gsm integrated spunlace nonwoven comprised of three carded strata having the following composition:

**Card 1 + Card 2 (37 gsm each):**

20% 1,7 dtex CV
40% 5,8 dtex PET/PE
40% 10 dtex PET HS

**Card 3 (36 gsm):**

80% 1,7 dtex CV
20% 10 dtex PET HS

The integrated material was heat-stiffened at a relatively low temperature range of 110°C - 115°C to provide integrity while keeping bendability of the resulting nonwoven. The following values were measured on Example 1:

HBD@100mm = 92 mm
C1 = 1.90 mm
C2 = 0.75 mm
C3 = 1.33 mm
Compliance Index = 78.9 mm$^3$/N
Percent Recovery = 70.0%

Example 2

Example 2 is a 110 gsm integrated spunlace nonwoven comprised of three carded strata having the following composition:

**Card 1 + Card 2 (37 gsm each):**

50% 5,8 dtex PE/PET
50% 10 dtex PET HS

**Card 3 (36 gsm):**

85% 1,7 dtex CV
15% 10 dtex PET HS

The following values were measured on Example 1:

HBD@100mm = 95 mm
C1 = 1.87 mm
C2 = 0.66 mm
C3 = 1.40 mm
Compliance Index = 83.0 mm$^3$/N
Percent Recovery =75.0%

While both examples 1 and 2 are suitable to be used in an absorbent article, example 1 is particular suitable as an acquisition layer as it also comprises absorbent fibers (CV) in all three strata. For both examples, it may be preferred that the stratum comprising the higher percentage of absorbent fibers (CV) be disposed towards the absorbent core, and the other side of the nonwoven towards the topsheet, to minimize rewet at the surface of the topsheet.

**Test Methods**

The values indicated herein are measured according to the methods indicated herein below, unless specified otherwise. All measurements are performed at 23°C ± 2°C and 50% ± 2% Relative Humidity, unless specified otherwise. All samples should be kept at least 24 hours in these conditions to equilibrate before conducting the tests, unless indicated otherwise. If possible, measurements are made on the component materials before they are integrated in an absorbent article. If this is not possible, care should be exerted when excising the sample to not impart any contamination or distortion to the test sample layer during the removal the material from other layers (using cryogenic spray, such as Cyto-Freeze, Control Company, Houston, Texas, if needed).

Horizontal Bending Drop at 100 mm (HBD@100mm) Measurement Method

**Principle:** this method measures the ability of a nonwoven material to bend under his own weight (sometimes designated as "drapability"). The measurement principle is to hang a length of 100 mm of the material over a sharp 90° edge and measure the vertical drop of this length of the material under its own weight, expressed in mm. This vertical drop is illustrated as reference number 1 in Fig. 1.

**Apparatus:** the setup for conducting the measurement is schematically shown in Fig. 1 and comprises:

i) a flat support box 2 made of any suitable material such as polycarbonate (e.g. Lexan®) about 400 mm long, about 200 mm wide and a height 3 of exactly 140 mm, with at least one of its top edge 4 in the width direction having a sharp 90 degree angle. The box 1 is positioned on a suitable flat surface 5, such as a lab bench,
ii) a movable vertical metal ruler 6, having a stable horizontal foot, and calibrated so that its zero corresponds to the flat surface 5 on which the box is disposed. The movable vertical metal ruler is used to measure the distance 7 of the hanging edge 8 of the material specimen 9 to the flat surface.

**Procedure:** a rectangular material specimen 9 having a width of about 80 mm and a length of about 200 mm is cut from a roll stock of the nonwoven. The length corresponds to the machine direction of the nonwoven and the width corresponds to the cross-direction of the nonwoven. The method can be alternatively conducted on a material specimen having a width of about 50 mm if the nonwoven's original width is shorter than 80 mm.

The material specimen 9 is laid flat on any suitable flat surface such as a lab bench, and a line is drawn at exactly at 100 mm from the front edge 8 of the material specimen in the width direction.

The material specimen 9 is then laid on top of the support box 2 with a first side of the specimen facing up (side A). The 100 mm line drawn is precisely positioned on the sharp edge 4 with the 100 mm long portion of the material specimen hanging free from the support box 2, as illustrated on Fig. 1.

The movable ruler 6 is positioned near the front edge 8 of the hanging specimen material so that the distance 7 of the hanging front edge 8 from the flat surface 5 can be measured. Since the hanging front edge 8 may not be perfectly horizontal, the distance is measured on the two corners of the hanging front edge 8, as well as in the center of the front edge 8, and the arithmetic mean of the three values recorded to the nearest mm.

The bending drop 1 is calculated as the difference between the exact Drape box height 3 (140 mm) and the recorded vertical distance 7 of the front edge 8 to the flat surface 5, as measured with the ruler 6 from the flat surface 5.

The material specimen is then turned upside down (side B now up), and the same procedure described above is performed. The bending drop recorded overall for the material specimen is the greater of the side A bending drop and the side B bending drop.

The overall procedure above is repeated on five like material specimens. The arithmetic mean of the bending drop values for the five like material specimens is reported to the nearest mm as the Horizontal Bending Drop at 100 mm (HBD@100mm) for the nonwoven tested.

Z-Compliance Index and Percent Recovery Measurement Method

**Principle:** this method measures the ability of a nonwoven material to be compressed in z-direction under applied pressure and then to recover to its original caliper after removing said applied pressure.

**Setup:** a vertically oriented electronic caliper tester having a precision of at least 0.01 mm with a 40 mm diameter circular foot may be used. The pressure exerted by the foot on the specimen is adjustable via the addition of pre-selected weights. Measurements are made at 0.85 $\pm$ 0.05 kPa and 15.5 $\pm$ 0.1kPa.

**Procedure:** A material specimen is cut from the nonwoven roll stock to a square sample with a width of about 80 mm (or alternatively in case the material is not available in the suitable size in a material specimen with a width of about 50 mm).

The square sample specimen is positioned centered under the caliper foot and the caliper at 0.85 $\pm$ 0.05 kPa (P1) is measured and recorded to the nearest 0.01 mm (C1). Without removing the sample from the equipment, the pressure is increased to 15.4 $\pm$ 0.1 kPa (P2) and the caliper measured and recorded to the nearest 0.01 mm (C2). The pressure may be increased by adding a suitable weight on the caliper foot. Again without moving the sample, the exerted pressure is reduced back to 0.85 $\pm$ 0.05 kPa (for example by removing the extra weight) and the caliper measured a third time (C3) and recorded to the nearest 0.01mm.

For the specimen being measured, the compliance index is defined as:

$$\text{compliance index} = (C1-C2) / (P2-P1)$$

and is recorded to the nearest 0.1 mm$^3$/N.

The recovery is calculated as:

$$\text{recovery} = C3 / C1 * 100\%$$

expressed in percent and recorded to the nearest 0.1%.

The procedure above is conducted on five like specimens of the same nonwoven material. The arithmetic mean of the compliance index values among the five specimens is calculated and reported to the nearest 0.1 mm$^3$/N as the Compliance Index. The arithmetic mean of percent recovery values among the five specimens is calculated and reported to the nearest 0.1 % as the Percent Recovery.

**General description of an absorbent article 20**

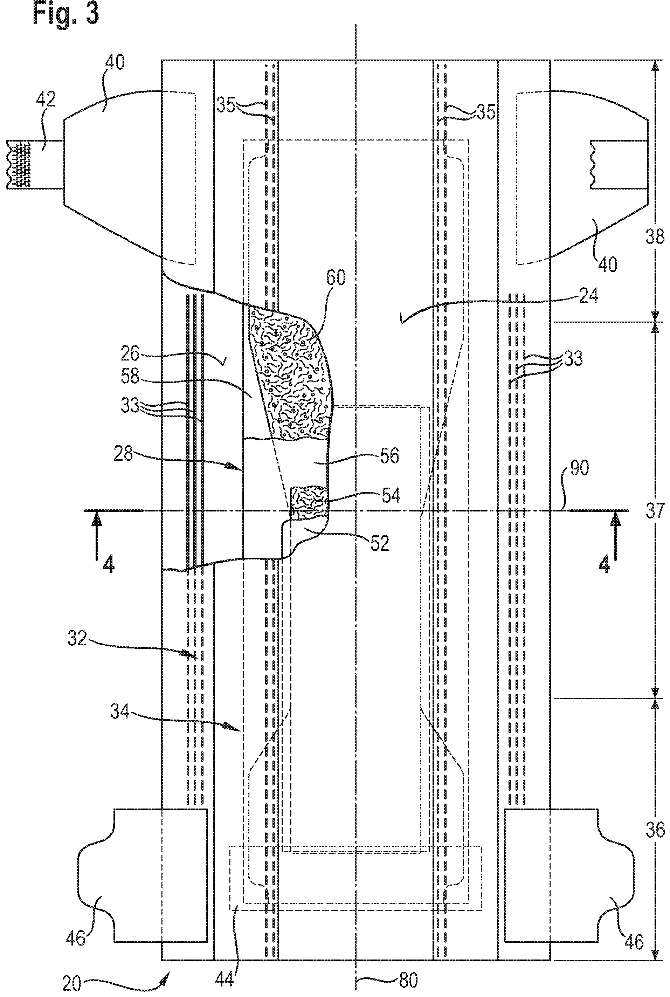

An exemplary absorbent article that may use a nonwoven of the invention is represented in Figs. 3-4 in the form of a baby taped diaper 20. This taped diaper 20 is shown for illustration purpose only as the invention is applicable to a wide variety of diapers or other absorbent articles such as baby diaper pants, adult incontinence pants or feminine sanitary pads. In the following, the word "diaper" and "absorbent article" are used interchangeably. The Figures are used herein as illustration of one way to carry out the invention and are not limiting the scope of the claims, unless specifically indicated to do so.

The absorbent article comprises a liquid permeable topsheet 24 on its wearer-facing surface, a liquid impermeable backsheet 25 on its garment-facing surface and an absorbent core 28 between the topsheet and the backsheet. The topsheet typically forms the majority of the wearer-contacting surface of the article and is the first layer that the body exudates contact. The topsheet is liquid permeable, permitting liquids to readily penetrate through its thickness. Any known topsheet may be used in the present invention. The backsheet typically comprises a fluid impermeable plastic film, which may be printed with a backsheet pattern, and a low basis weight nonwoven cover glued to this impermeable film to give a nicer feel and appearance to the backsheet.

The absorbent article has a longitudinal axis 80 extending longitudinally from the front to the back of the article and notionally dividing the article into a left and right half. The length L of the article may be measured along the longitudinal axis 80. The absorbent article can also be notionally divided by a transversal axis 90 at half the length L. The article can be further notionally divided in three regions having equal length of a third of L along the longitudinal axis: a front region 36 extending from the front edge towards the crotch region for a third of L, a crotch region 37 in the middle third of the diaper, and a back region 38 extending from the crotch region to the back edge of the article for the remaining third of L. All three regions are of equal length measured on the longitudinal axis, when the article is in such a flat state. The front region, crotch region, back region and longitudinal and transversal axis are defined herein notionally, that is they are typically not materialized in the real diapers, but are useful to describe the positions of various components of the invention relative to each other and the diaper.

The absorbent article may also comprise a fluid acquisition layer 54, which may be advantageously a nonwoven as previously disclosed, between the topsheet and the absorbent core. Other typical diaper components include elasticized gasketing cuffs 32 and upstanding barrier leg cuffs 34, which are present in most diapers. The absorbent article may also comprise other known diaper components, which are not represented in the Figures, such as transverse barrier cuffs, front and/or back elastic waistbands, a lotion application on the topsheet, longitudinally extending channels in the core and/or the distribution layer, a wetness indicator, etc... all these components have been described and exemplified in the art and are not further detailed herein. More detailed disclosures of example of such components are for example disclosed in WO201493323, WO2015/183669 (both Bianchi et al), WO 2015/031225 (Roe et al.) or WO2016/133712 (Ehrnsperger et al.) to name a few.

The topsheet 24, the backsheet 25, the absorbent core 28, the nonwoven of the invention and other article components may be assembled in a variety of well-known configurations, in particular by gluing, fusion and/or pressure bonding. The absorbent articles of the invention may comprise any typical layers and components used in absorbent products of the diaper type, and which are not necessarily represented in the simplified Figs. 3-4. The invention thus also encompasses a process for making an absorbent article comprising the steps of combining a nonwoven according to the present invention with the other absorbent article components, e.g. topsheet, backsheet and absorbent core.

**General description of an absorbent core 28**

The absorbent core 28 is the component of the absorbent article having the most absorbent capacity and comprises an absorbent material layer 60. The absorbent material layer 60 may be generally rectangular or may be sand-hour shaped, defining a tapering along its width towards the middle region of the core as shown in Fig. 3. This absorbent core represented is of course not limiting the scope of the invention as the invention is applicable to a wide variety of absorbent cores. In this way, the absorbent material deposition area may have a relatively narrow width in an area of the core intended to be placed in the crotch region of the absorbent article. This may provide for example better wearing comfort. Other shapes can also be used such as rectangular, a "T" or "Y" or "dog-bone" shape for the area of the absorbent material.

The absorbent material 60 may be any conventional absorbent material known in the art. For example, the absorbent material may comprise a blend of cellulose fibers and superabsorbent particles 66 ("SAP"), typically with the percentage of SAP ranging from 40% to 70% by weight of the absorbent material. The absorbent material layer 60 may also be free of cellulose fibers, as is known in so-called airfelt-free cores where the absorbent material consists of SAP. Airfelt-free cores are typically much thinner and more flexible compared to conventional cores that comprise cellulose fibers. Cellulose-free absorbent cores may thus be particularly useful with an acquisition layer made of a nonwoven according to the present invention.

"Superabsorbent polymers" or "SAP" as used herein refer to absorbent material which are cross-linked polymeric materials that can absorb at least 10 times, preferably at least 15 times, their weight of an aqueous 0.9% saline solution as measured using the Centrifuge Retention Capacity (CRC) test (EDANA method WSP 241.2-05E). These polymers are typically used in particulate forms so as to be flowable in the dry state. The term "particles" refers to granules, fibers, flakes, spheres, powders, platelets and other shapes and forms known to persons skilled in the art of superabsorbent polymer particles.

Various absorbent core designs comprising high amount of SAP have been proposed in the past, see for example in US5,599,335 (Goldman), EP1,447,066 (Busam), WO95/11652 (Tanzer), US2008/0312622A1 (Hundorf), WO2012/052172 (Van Malderen). In particular the SAP printing technology as disclosed in US2006/024433 (Blessing), US2008/0312617 and US2010/0051166A1 (both to Hundorf et al.) may be used. The invention is however not limited to a particular type of absorbent core. The absorbent core may also comprise one or more glue such as auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity.

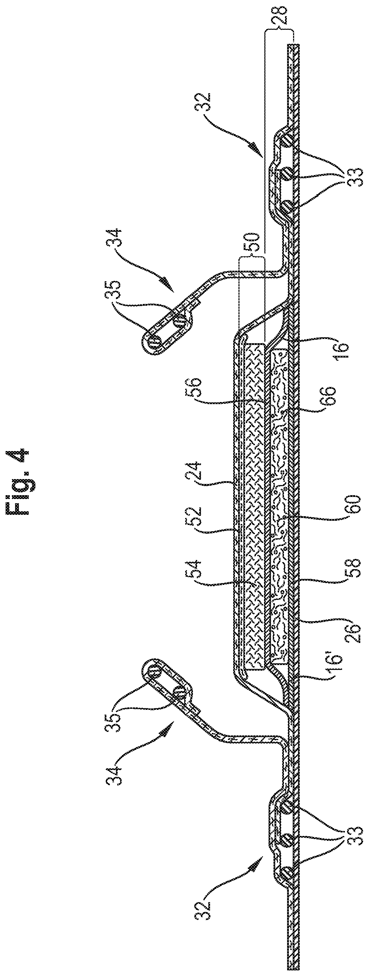

The absorbent material may be for example deposited as a continuous layer between the top layer 16 and the bottom layer 16' of the core wrap. The core wrap is typically a low basis weight nonwoven, e.g. a SMS material (spunbond-meltblown-spunbond laminate). The top layer and bottom layer may be attached (e.g. by gluing) at their longitudinal edges in a face to face relationship as shown in Fig. 4, or alternatively in a C-wrap wherein one layer is larger than the other layer, having a pair of flaps that are folded over the other layer as is known in the art. The absorbent material may also be present discontinuously for example as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern wherein the resulting layer is substantially continuously distributed across the absorbent particulate polymer material area. As for example taught in US2008/0312622A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core.

The absorbent core may also comprise longitudinally extending channels (not represented) which are areas substantially free of absorbent material within the absorbent material layer 60. The core wrap may be bonded through these material-free areas. Exemplary disclosures of such channels in an airfelt-free core can be found in WO2012/170778 (Rosati et al.) and US2012/0312491 (Jackels). Channels may of course also be formed in absorbent cores comprising cellulose fibers.

## Acquisition layer 54

As indicated previously, the nonwoven of the present information can be advantageously used as an acquisition layer 54 disposed between the topsheet 24 and the absorbent core 28. The acquisition layer 54 may have any suitable size, and may be smaller, larger or same size as the absorbent material layer 60. The article may also comprise an additional acquisition layer 52, for example between the acquisition layer of the invention and the topsheet. Such additional acquisition layer 52 may in particular be an air-through bonded carded nonwoven, for example having a basis weight of from 20 gsm to 100 gsm, or from 30 gsm to 80 gsm. Such additional acquisition layer is typically made of synthetic fibers that have been hydrophillically treated with a surfactant. The first and second acquisition layers may form together an acquisition system 50.

## Packages

Absorbent articles for personal hygiene are typically packaged by the manufacturer in a plastic bag and/or a cardboard box for transport and sale. The articles may also be folded before being packaged to save space as is known in the art. The back and front ears of taped diapers are for example typically folded inwardly before bi-folding the diaper along its transversal axis before being packaged. The absorbent articles may be packaged under compression, so as to reduce the size of the package so that the caregivers can easily handle and store the packages, while also providing distribution and inventory savings to manufacturers owing to the size of the packages. The package may for example comprise from 2 to 200 of the articles.

## Claims

1. A nonwoven suitable for use in an absorbent article, wherein the nonwoven has a basis weight ranging from about 40 grams per square meter (gsm) to about 200 gsm and is an integrated, carded, staple fiber nonwoven comprising a plurality of absorbent fibers, a plurality of stiffening fibers and a plurality of resilient fibers, the nonwoven comprising, by weight of the nonwoven:

   - from about 20 percent to about 75 percent of absorbent fibers;
   - from about 1 percent to about 50 percent of stiffening fibers;
   - from about 10 percent to about 50 percent of resilient fibers:
   wherein the nonwoven has the following properties:

   - a Horizontal Bending Drop at 100 mm of at least 75 mm, preferably at least 80 mm, and more preferably at least 85 mm, as measured with the Horizontal Bending Drop at 100 mm Measurement Method described herein; and
   - a Z-Compliance Index of at least 50 $mm^3/N$, preferably at least 60 $mm^3/N$, as measured according to the Z-Compliance Index and Percent Recovery Measurement Method described herein.

2. A nonwoven according to claim 1, wherein the nonwoven has a thickness (C1) of from 0.50 mm to 4.00 mm, as measured at a pressure of 0.85 kPa according to the Z-Compliance Index and Percent Recovery Measurement Method described herein.

3. A nonwoven according to claim 2, wherein the nonwoven has a thickness (C1) of from 1.00 mm to 3.00 mm.

4. A nonwoven according to any of the preceding claims, wherein the nonwoven has a basis weight ranging from about 70 gsm to about 120 gsm.

5. A nonwoven according to any of the preceding claims, wherein the nonwoven has a Percent Recovery of at least 50%, as measured by the Z-Compliance Index and Percent Recovery Measurement Method described herein.

6. A nonwoven according to the preceding claim, wherein the nonwoven has a Percent Recovery of at least 60%, more preferably at least 70% as measured by the Z-Compliance Index and Percent Recovery Measurement Method described herein.

7. A nonwoven according to any of the preceding claims, wherein the nonwoven is an integrated nonwoven that comprises at least two integrated, carded strata, in particular three or more integrated, carded strata (214, 224, 234).

8. A nonwoven according to claim 7, wherein each stratum comprises absorbent fibers.

9. A nonwoven according to claim 7 or 8, wherein the integrated, carded, staple fiber nonwoven has a first side and a second side, and wherein there is a higher amount of absorbent fibers on one side versus the other side.

10. An absorbent article (20) comprising a topsheet (24), a backsheet (26), an absorbent core (28) and a nonwoven (54) according to any of the preceding claims.

11. An absorbent article (20) according to claim 10, wherein the absorbent core comprises superabsorbent polymers (66).

12. An absorbent article (20) according to claim 10 or 11, wherein the absorbent core does not comprise cellulose fibers mixed with the superabsorbent polymers.

13. An absorbent article (20) according to any of claim 10 - 12, wherein the absorbent article comprises an acquisition layer (54) between the topsheet and the absorbent core (28), and the acquisition layer is a nonwoven according to any of the preceding claims 1-9.

14. An absorbent article according to claim 13, wherein the absorbent article comprises an additional acquisition layer (52) between the topsheet and the acquisition layer (54).

15. An absorbent article according to claim 14, wherein the additional acquisition layer (52) is an air-through bonded carded nonwoven having a basis weight of from 20 gsm to 100 gsm.

**Patentansprüche**

1. Vlies, das zum Gebrauch in einem Absorptionsartikel geeignet ist, wobei das Vlies ein Basisgewicht in einem Bereich von etwa 40 Gramm pro Quadratmeter (g/m$^2$) bis etwa 200 g/m$^2$ aufweist und ein integriertes, kardiertes Stapelfaservlies ist, umfassend eine Vielzahl von Absorptionsfasern, eine Vielzahl von Versteifungsfasern und eine Vielzahl von elastischen Fasern, das Vlies umfassend:

   - zu etwa 20 Gewichtsprozent bis etwa 75 Gewichtsprozent Absorptionsfasern;
   - zu etwa 1 Gewichtsprozent bis etwa 50 Gewichtsprozent Versteifungsfasern;
   - zu etwa 10 Gewichtsprozent bis etwa 50 Gewichtsprozent elastische Fasern:
   wobei das Vlies die folgenden Eigenschaften aufweist:

   - einen horizontalen Biegeabfall bei 100 mm von mindestens 75 mm, vorzugsweise mindestens 80 mm und mehr bevorzugt mindestens 85 mm, gemessen mit dem hierin beschriebenen Messverfahren für den horizontalen Biegeabfall bei 100 mm; und
   - einen Z-Konformitätsindex von mindestens 50 mm$^3$/N, vorzugsweise mindestens 60 mm$^3$/N, gemessen gemäß dem hierin beschriebenen Messverfahren für den Z-Konformitätsindex und die prozentuale Rückstellung.

2. Vlies nach Anspruch 1, wobei das Vlies eine Dicke (C1) von 0,50 mm bis 4,00 mm aufweist, gemessen bei einem Druck von 0,85 kPa gemäß dem hierin beschriebenen Messverfahren für den Z-Konformitätsindex und die prozentuale Rückstellung.

3. Vlies nach Anspruch 2, wobei das Vlies eine Dicke (C1) von 1,00 mm bis 3,00 mm aufweist.

4. Vlies nach einem der vorstehenden Ansprüche, wobei das Vlies ein Basisgewicht in einem Bereich von etwa 70 g/m$^2$ bis etwa 120 g/m$^2$ aufweist.

5. Vlies nach einem der vorstehenden Ansprüche, wobei das Vlies eine prozentuale Rückstellung von mindestens 50 % aufweist, gemessen durch das hierin beschriebene Messverfahren für den Z-Konformitätsindex und die prozentuale Rückstellung.

6. Vlies nach dem vorstehenden Anspruch, wobei das Vlies eine prozentuale Rückstellung von mindestens 60 %, mehr bevorzugt mindestens 70 % aufweist, gemessen durch das hierin beschriebene Messverfahren für den Z-Konformitätsindex und die prozentuale Rückstellung.

**7.** Vlies nach einem der vorstehenden Ansprüche, wobei das Vlies ein integriertes Vlies ist, das mindestens zwei integrierte, kardierte Lagen, insbesondere drei oder mehr integrierte, kardierte Lagen (214, 224, 234), umfasst.

**8.** Vlies nach Anspruch 7, wobei jede Lage Absorptionsfasern umfasst.

**9.** Vlies nach Anspruch 7 oder 8, wobei das integrierte, kardierte Stapelfaservlies eine erste Seite und eine zweite Seite aufweist und wobei sich auf einer Seite eine größere Menge von Absorptionsfasern befindet als auf der anderen Seite.

**10.** Absorptionsartikel (20), umfassend eine Oberschicht (24), eine Unterschicht (26), einen Absorptionskern (28) und ein Vlies (54) nach einem der vorstehenden Ansprüche.

**11.** Absorptionsartikel (20) nach Anspruch 10, wobei der Absorptionskern Superabsorber-Polymere (66) umfasst.

**12.** Absorptionsartikel (20) nach Anspruch 10 oder 11, wobei der Absorptionskern keine Cellulosefasern umfasst, die mit den Superabsorber-Polymeren vermischt sind.

**13.** Absorptionsartikel (20) nach einem der Ansprüche 10 bis 12, wobei der Absorptionsartikel eine Aufnahmeschicht (54) zwischen der Oberschicht und dem Absorptionskern (28) umfasst und die Aufnahmeschicht ein Vlies nach einem der vorstehenden Ansprüche 1 bis 9 ist.

**14.** Absorptionsartikel nach Anspruch 13, wobei der Absorptionsartikel eine zusätzliche Aufnahmeschicht (52) zwischen der Oberschicht und der Aufnahmeschicht (54) umfasst.

**15.** Absorptionsartikel nach Anspruch 14, wobei die zusätzliche Aufnahmeschicht (52) ein luftdurchleitungsgebundenes, kardiertes Vlies ist, das ein Basisgewicht von 20 g/m$^2$ bis 100 g/m$^2$ aufweist.

## Revendications

**1.** Non-tissé approprié pour une utilisation dans un article absorbant, dans lequel le non-tissé a un poids de base allant d'environ 40 grammes par mètre carré (g/m$^2$) à environ 200 g/m$^2$ et est un non-tissé en fibre discontinue cardé intégré comprenant une pluralité de fibres absorbantes, une pluralité de fibres de raidissement et une pluralité de fibres élastiques, le non-tissé comprenant, en poids du non-tissé :

- d'environ 20 pour cent à environ 75 pour cent de fibres absorbantes ;
- d'environ 1 pour cent à environ 50 pour cent de fibres de raidissement ;
- d'environ 10 pour cent à environ 50 pour cent de fibres élastiques :
dans lequel le non-tissé a les propriétés suivantes :

- une chute de cintrage horizontale à 100 mm d'au moins 75 mm, de préférence d'au moins 80 mm, et plus préférablement d'au moins 85 mm, telle que mesurée selon le procédé de mesure de la chute de cintrage horizontale à 100 mm décrit ici ; et
- un indice de conformité Z d'au moins 50 mm$^3$/N, de préférence d'au moins 60 mm$^3$/N, mesuré selon le procédé de mesure de l'indice de conformité Z et du pourcentage de récupération décrit ici.

**2.** Non-tissé selon la revendication 1, dans lequel le non-tissé a une épaisseur (C1) de 0,50 mm à 4,00 mm, telle que mesurée à une pression de 0,85 kPa selon le procédé de mesure de l'indice de conformité Z et du pourcentage de récupération décrit ici.

**3.** Non-tissé selon la revendication 2, dans lequel le non-tissé a une épaisseur (C1) de 1,00 mm à 3,00 mm.

**4.** Non-tissé selon l'une quelconque des revendications précédentes, dans lequel le non-tissé a un poids de base allant d'environ 70 g/m$^2$ à environ 120 g/m$^2$.

**5.** Non-tissé selon l'une quelconque des revendications précédentes, dans lequel le non-tissé a un pourcentage de récupération d'au moins 50 %, tel que mesuré par le procédé de mesure de l'indice de conformité Z et du pourcentage de récupération décrit ici.

**6.** Non-tissé selon la revendication précédente, dans lequel le non-tissé a un pourcentage de récupération d'au moins 60 %, plus préférablement d'au moins 70 %, tel que mesuré par le procédé de mesure de l'indice de conformité Z et du pourcentage de récupération décrit ici.

**7.** Non-tissé selon l'une quelconque des revendications précédentes, dans lequel le non-tissé est un non-tissé intégré qui comprend au moins deux strates cardées intégrées, en particulier trois strates cardées intégrées (214, 224, 234) ou plus.

**8.** Non-tissé selon la revendication 7, dans lequel chaque strate comprend des fibres absorbantes.

**9.** Non-tissé selon la revendication 7 ou 8, dans lequel le non-tissé en fibre discontinue cardé intégré a un premier côté et un second côté, et dans lequel il y a une plus grande quantité de fibres absorbantes sur un côté par rapport à l'autre côté.

**10.** Article absorbant (20) comprenant une feuille de dessus (24), une feuille de fond (26), un noyau absorbant (28) et un non-tissé (54) selon l'une quelconque des revendications précédentes.

**11.** Article absorbant (20) selon la revendication 10, dans lequel le noyau absorbant comprend des polymères superabsorbants (66).

**12.** Article absorbant (20) selon la revendication 10 ou 11, dans lequel le noyau absorbant ne comprend pas de fibres de cellulose mélangées aux polymères superabsorbants.

**13.** Article absorbant (20) selon l'une quelconque des revendications 10 à 12, dans lequel l'article absorbant comprend une couche d'acquisition (54) entre la feuille de dessus et le noyau absorbant (28), et la couche d'acquisition est un non-tissé selon l'une quelconque des revendications précédentes 1 à 9.

**14.** Article absorbant selon la revendication 13, dans lequel l'article absorbant comprend une couche d'acquisition supplémentaire (52) entre la feuille de dessus et la couche d'acquisition (54).

**15.** Article absorbant selon la revendication 14, dans lequel la couche d'acquisition supplémentaire (52) est un non-tissé cardé lié à l'air ayant un poids de base de 20 g/m$^2$ à 100 g/m$^2$.

Fig. 1

Fig. 2

**Fig. 3**

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180098889 A1
- US 20140343523 A1
- US 6333108 B
- US 5634914 A
- US 5458835 A, Wilkes
- US 7767598 B, Schneider
- WO 9900098 A
- WO 201493323 A
- WO 2015183669 A, Bianchi
- WO 2015031225 A, Roe
- WO 2016133712 A, Ehrnsperger

- US 5599335 A, Goldman
- EP 1447066 A, Busam
- WO 9511652 A, Tanzer
- US 20080312622 A1, Hundorf
- WO 2012052172 A, Van Malderen
- US 2006024433 A, Blessing
- US 20080312617 A
- US 20100051166 A1, Hundorf
- WO 2012170778 A, Rosati
- US 20120312491 A, Jackels